Europäisches Patentamt

European Patent Office

Office européen des brevets

(11) Publication number: **0 103 620**
**B1**

(12) **EUROPEAN PATENT SPECIFICATION**

(45) Date of publication of patent specification: **17.08.88**

(21) Application number: **83901088.1**

(22) Date of filing: **18.03.83**

(86) International application number:
**PCT/GB83/00081**

(87) International publication number:
**WO 83/03477 13.10.83 Gazette 83/24**

(51) Int. Cl.⁴: **G 01 N 33/80**

(54) **BLOOD CELL GROUPING REAGENT.**

(30) Priority: **29.03.82 GB 8209235**
**25.02.83 GB 8305364**

(43) Date of publication of application:
**28.03.84 Bulletin 84/13**

(45) Publication of the grant of the patent:
**17.08.88 Bulletin 88/33**

(84) Designated Contracting States:
**AT BE CH DE FR LI LU NL SE**

(56) References cited:
**WO-A-81/02104**
**WO-A-82/03089**

**Chemical Abstracts, vol. 97, no. 11, 13
September 1982 (Columbus, Ohio, US) D. Voak
et al.: "Monoclonal anti-A and anti-B:
development as cost effective reagents", see
page 587, column 2, abstract no. 90128c, Med.
Lab. Sci. 1982, 39(2), 109-22**

(73) Proprietor: **CELLTECH LIMITED**
**244-250 Bath Road**
**Slough Berkshire SL1 4DY (GB)**

(72) Inventor: **LENNOX, Edwin Samuel**
**Fleam Dyke House 25 Frog End**
**Great Wilbraham Cambridgeshire (GB)**
Inventor: **VOAK, Douglas**
**10 The Coppice Impington**
**Cambridge CB4 4PP (GB)**

(74) Representative: **Votier, Sidney David et al**
**CARPMAELS & RANSFORD 43, Bloomsbury
Square**
**London WC1A 2RA (GB)**

(56) References cited:
**Biological Abstracts, vol. 72, no. 2, published in
1981 (Philadelphia, US) D. Voak et al.:
"Monoclonal anti-A from a hybrid myloma:
evaluation as a blood grouping reagent", see
page 1034, column 2, abstract no. 9903, Vox
Sang. 39(3), 1980, 134-140**

Courier Press, Leamington Spa, England.

**0 103 620**

(56) References cited:

Vox Sanguinis, vol. 40, published in 1981, S.H. Sarks et al.: "Monoclonal anti-B as a new blood-typing reagent", see page 99-104

The Journal of Immunology, vol. 129, no. 2, published in August, 1982 (Baltimore, US) D.R. Bundle et al.: "Hybridomas specific for carbohydrates; synthetic human blood group antigens for the production, selection and characterization of monoclonal typing reagents", see pages 678-682

Medical Laboratory Sciences, vol. 36, published in 1979, A.D. Blann: "Cell hybrides: an important new source of antibody production", see pages 329-338

Clinical Chemistry, vol. 27, no. 11, published in November 1981 (Easton, US) E.D. Sevier et al.: "Monoclonal antibodies in clinical immunology", see pages 1797-1806

## Description

The present invention relates to ABO blood cell grouping reagents.

ABO incompatibility is a potentially lethal barrier in transfusion therapy and in tissue transplantation. All donor blood and potential recipients for transfusion or tissue transplantation are therefore routinely ABO grouped as this is the only way to ensure maximum safety for these patients.

ABO blood cell grouping is conventionally performed by testing the red cells of the blood to be typed for the presence or absence of antibodies and antigens. The presence of the latter is generally determined by adding an anti-serum (a blood serum containing known antibodies to the A and B red cell antigens) to the red cells in saline suspension and looking for agglutination. The anti-sera employed in the grouping of ABO blood contain either anti-A antibodies (Group B serum) or anti-B antibodies (Group A serum) or both anti-A and anti-B antibodies (Group O serum). Of these, Group O serum is used to check that the anti-A and anti-B tests, that are used in all routine ABO groupings of blood donations and patients, are correct.

The main factors affecting the choice of ABO reagents are,

(a) The specificity of the reagent for the appropriate antigen.

(b) The stability of the reagent under the conditions of use.

(c) The potency or agglutinating quality which must be sufficient to give reliable results by the routine methods employed.

(d) The guaranteed availability of sufficient volumes to meet the commitments of the transfusion service, and

(e) The cost of production which must be as low as possible.

At the present the National Health Service (NHS) obtains anti-sera either by screening the sera of donor blood samples or by hyperimmunisation of volunteer donors. By contrast commercial sources and most European and North American countries obtain sera only from hyperimmunised donors. In both cases the supply of potent group O serum is limited, and in the latter case expensive. This limited availability cannot be remedied by blending the more readily obtainable group A and group B sera, since this leads to the loss of some anti-A and anti-B activity by cross neutralisation.

It has recently been published that anti-A and anti-B sera can be obtained individually in the form of monoclonal antibodies. Voak et al (Vox. Sang. 39, 134—140 (1980)) disclose the preparation of monoclonal anti-A antibodies as blood group reagents, and Sacks et al (Vox. Sang. 40, 99—104 (1981)) similarly disclose the preparation of monoclonal anti-B antibodies as blood group reagents. However, neither publication suggests a solution to the above problems associated with the preparation of group O serum.

It is the aim of the present invention to provide a new ABO blood cell grouping reagent that may be used in place of conventional group O serum and that is both readily available and cheap to produce.

According to the present invention there is provided an ABO blood cell grouping reagent comprising, in admixture, one or more anti-A monoclonal antibodies and one or more anti-B monoclonal antibodies.

The present ABO blood cell grouping reagent is an anti-A/B grouping reagent which detects A antigenic determinants and B antigenic determinants on red blood cells.

In the present specification an anti-A monoclonal antibody incorporates an anti-A,B monoclonal antibody, provided the anti-A,B monoclonal antibody is potent enough to cause a macroscopic reaction (by spin-tube, tube sedimentation, or tile/slide methods) with red blood cells possessing one or more A-type antigenic determinants. Moreover, an anti-B monoclonal antibody incorporates an anti-A,B monoclonal antibody, provided the anti-A,B monoclonal, antibody is potent enough to cause a macroscopic reaction (by spin-tube, tube sedimentation or tile/slide methods) with red blood cells possessing one or more B-type antigenic determinants.

Any mixture of monoclonal anti-A and anti-B antibodies that has sufficiennt agglutinating quality to give reliable results (for the presence of antigens A and B) by the routine blood typing methods may be used as an ABO reagent according to this invention. A 'sufficient agglutinating quality' will be an agglutinating quality as good as or better than that displayed by current UK National Health Service group O reagents. 'Routine blood typing methods' are set out in full in "Techniques in Blood Groupings" by I Dunsford and C C Bowley, 2nd Edn., 1967.

In a further aspect of the present invention the reagent may also incorporate one or more monoclonal antibodies (in particular monoclonal anti-A,B antibodies) that are specific for the antigenic determinants on Ax-type red blood cells (Ax activity) and Bx-type red blood cells (Bx activity) (or other weak B variants). A reagent that possesses Ax activity is particularly useful since such a reagent should prevent the mistyping of certain weak A-type blood cells as group O.

Preferably the monoclonal antibodies are derived from hybrid myelomas produced by the fusion of mouse myeloma cells, especially NSI (abbreviated from P3-NSI/1-Ag4-1, G Kohler, S C Howe and C Milstein, Eur J Immunol, 1976, 6, 292) or NSO/1 (G Galfre and C Milstein, Methods in Enzymology, 1981, 73, 3), both available on request from the MRC Laboratory of Molecular Biology, Hills Road, Cambridge), with spleen or other immunocyte cells from mice immunised with human antigen A, human antigen B, or human antigen A,B. In one particularly preferred embodiment of monoclonal anti-A (6D4) and a monoclonal anti-B (NB1/19), derived respectively from the hybrid myeloma 6D4. 12.3 and NB1/19.112.28 are mixed. In another preferred embodiment a monoclonal anti-A (3D3), derived from the hybrid myeloma A15.3D3. 92. OO, and monoclonal anti-B (NB1/19) are mixed.

3

The monoclonal antibodies used to form the present reagent may be separated either from the ascitic fluids of tumours produced in animals, generally mice, or from tissue culture supernatants.

It will be seen from the above that the present reagent is not an anti-serum as previously understood since the antibodies are not contained in a serum. However the medium surrounding the monoclonal antibodies will still, in many cases, contain a certain proportion of serum. In the former case the serum will often be present in the ascitic fluid as collected, whilst in the latter case the serum will be added to the tissue culture to provide nutrients for cell growth.

In addition to this serum the present reagent may also contain a certain proportion of conventional Group O serum. The addition of this serum is to provide the present reagent with $A_x$ activity, especially in cases where the mixture of monoclonal antibodies alone lacks this activity. In a particularly preferred embodiment a one-third volume of Group O serum is added.

The present reagent will be used primarily to check that anti-A and anti-B tests (for antigens A and B) are correct. In order to facilitate this test there is also provided a process for the grouping of ABO blood cells comprising adding to a suspension of cells in sodium chloride solution, a blood cell grouping reagent which comprises, in admixture, one or more anti-A monoclonal antibodies and one or more anti-B monoclonal antibodies. Once again the terms anti-A and anti-B monoclonal antibodies incorporate certain anti-A,B monoclonal antibodies, as defined above. Moreover the preferred monoclonal antibodies for use in this process are also listed above. Further, the reagent may also contain a certain proportion of conventional group O serum, especially if none of the monoclonal antibodies employed exhibits Ax activity.

The ABO blood cell blended grouping reagents of the present invention are as stable as the individual antibodies used in the blend when stored at 4°C or lower temperatures. Once the present blood cell grouping reagent has been added to the blood cell suspension in sodium chloride solution, the cell grouping test is generally continued by observing whether or not agglutination of the cells takes place. Generally, if the present reagent contains an antibody specific for one or more of the antigens of the cells under test, then agglutination will take place. Such agglutination is a visible reaction and therefore may be followed visually in a qualitative or quantitative manner. On the other hand, if the present reagent does not contain an antibody specific for the blood cells antigens then generally, unless a cross reaction takes place, no agglutination will occur. (Cross reaction refers to both false reactions and auto antibody sensitised cells).

For convenience, the present reagent may be provided for use in the above process in the form of a blood cell grouping kit. In one embodiment of this kit the anti-A monoclonal antibodies and the anti-B monoclonal antibodies are provided in the form of a mixture. In a second embodiment the anti-A monoclonal antibodies and the anti-B monoclonal antibodies are provided in separate containers together with instructions on the method of mixing. In a third embodiment either of the previous kits also contains group O serum, either in admixture with or separate from the monoclonal antibodies.

The reagent, process and kit of the present invention will now be described by way of example only.

Materials and methods

Analysis to determine antibody heavy and light chains

The myeloma NS1 produces K light chains which, although not secreted by the myeloma, can be 'mixed' with the light chains of spleen cell origin and the antibody secreted as mixed chain types called HLK. Full antibody activity requires both light and heavy chains, hence HLK antibody molecules are defective as some of the light chains are non-antibody myeloma K chains and thus reduce the antibody binding efficiency of the molecules. Therefore it is obviously desirable to select HL variants to obtain the best agglutinating antibodies. Recently myelomas which do not produce K chains, e.g. NSO/1, have become available and this eliminates the HLK problem. Composition is established by radioautograph of electrophoretic runs detecting the $^{14}$C-lysine incorporated into the antibodies secreted by the hybridmyeloma cells. There are two types of procedure for separating the antibody compartments in polyacrylamide gel slabs for subsequent radioautograph analysis:

(i) Iso-electric focusing (IEF). The separation is based on pH gradient separating components having different iso-electric points with prior reduction by 2-mercapto ethanol to break the interchain links.

(ii) Electrophoresis of the reduced antibodies with sodium dodecyl sulphate polyacrylamide slab gel electrophoresis (SDS-PAGE). The separation is based on molecular sieving of the different sized components that have a similar negative charge by being coated with SDS.

Serum reagents

NHS anti-sera prepared by the Blood Group Reference Laboratory (BGRL) from sera referred by regional transfusion centres. Commercial antisera from several sources.

Hemagglutination tests

These were tube tests as set out in Dunsford and Bowley. Red cells were from ACD or clotted samples received in the transfusion centre. Cells were washed ×4 in isotonic saline for comparative titration studies, but otherwise used by preparing a 2—3% suspension in saline. Cells were used at 20% for slide tests and 5% for spin tube tests.

**0 103 620**

Antibody dilutions were made in saline and titration results scored.

Elution was by the heat method, and enhancement tests used 2% pre-papainised cell suspensions or 20% bovine albumin by the displacement method.

Example 1. Preparation of monoclonal anti-A (6D4)

C3H/He-mg mice (OLAC) were immunised with tissue culture cells (HT-29 from Dr J Fogh, Sloan Kettering Institute) from a human group A colon carcinoma case. Spleen cells ($10^8$) from the immunised mice were fused with mouse myeloma cells ($10^7$) by use of the polyethylene glycol method (Weir, Handbook of Experimental Immunology, 3rd Edn. vol II). The myeloma cells were non-secretor P3-NS1/1-Ag4-1 selected enzyme deficient cells that die in HAT medium.

The culture supernatants were tested 2 weeks post fusion and those cells with antibody binding activity on HT-29 cells were cloned on soft agar and grown to larger volumes, recloned, regrown and divided into aliquots for larger scale antibody production and liquid nitrogen storage of the selected cell lines.

The established anti-A secreting clone (6D4) was grown in Dulbecco's modified Eagle's medium (DMEM, Gibco Biocult) supplemented with foetal calf serum (FCS, Sera-Lab). The concentration of FCS is gradually reduced from 15 to 5%. The supplemented tissue culture medium was free of anti-A or anti-B activity and A and B substances. (antigens).

The tissue culture supernatants were centrifuged to remove cells and debris, supplemented with 10 mM Hepes and 0.1% sodium azide and stored at 4°, −20° or −40°C. Aliquots were concentrated 5 fold, 8 fold and 18 fold by ultra-filtration on an Amicon filter (PM 10) and stored at 4° and −20°C.

Example 2. Preparation of monoclonal anti-A (3D3)

Male B10 BR mice (QLAC) were immunised with $A_1$ cells followed by several injections of human blood group A substance. Spleen cells ($10^8$) from the immunised mice were fused with mouse myeloma cells ($10^7$) by the use of the polyethylene glycol method. The myeloma cells were non-secretor NS0/1 derived from P3-NS1/1/Ag4/1 selected enzyme deficient cells that die in HAT medium.

The culture supernatants were tested, beginning 2 week post fusion and those cells producing anti-A were selected by agglutination tests, then cloned on soft agar, grown to large volumes, recloned, regrown and divided into aliquots for larger scale antibody production and liquid nitrogen storage of the selected cell lines.

To prepare culture supernatants for testing, the established anti-A secreting clone (3D3) was grown in Dulbecco's modified Eagle's medium (DMEM, Gibco Biocult), supplemented with foetal calf serum (FCS, Serolab). The concentration of FCS was gradually reduced from 15% to 2.5%.

The supplemented tissue culture medium was free of anti-A or anti-B activity and A or B substances.

Example 3. Preparation of monoclonal anti-B (NB1—19).

To find suitable mice, serum samples were assayed for anti-B activity after absorption with group O cells to remove anti-species antibodies. A mouse with an anti-B agglutination titre of 1:8 after absorption was chosen and injected intraperitoneally with 100 µg group B substance (donated by Dr W Watkins, MRC Clinical Research Centre, Harrow) in 0.1 ml complete Freund's adjuvant (Difco Bacto). This injection was repeated after 5 weeks and boosted after a further 9 weeks with 200 µg B substance in 0.1 ml saline injected intravenously. 3 days later, the spleen was removed and a cell suspension was prepared.

Spleen cells ($10^8$) were fused with mouse myeloma NS1 cells ($10^7$) using polyethylene glycol. Growing cell hybrids were selected by their ability to produce specific anti-B activity, detected in the culture supernatant by agglutination assays (I Dunsford and C C Bowley, Techniques in blood grouping, 2nd Edn. 1967) using human A, B and O erythrocytes. Anti-B secreting hybrids were cloned twice on soft agar, and grown up into 1 litre spinner cultures in Dulbecco's Modified Eagle's Medium (DMEM, Gibco Biocult) supplemented with 5% (v/v) foetal calf serum (FCS, Sera-Lab). The cloned hybrids were stored in liquid nitrogen.

Tissue culture supernatant containing the monoclonal antibody was prepared by centrifugation to remove cells and debris, filtration through Millipore® filters and addition of 10 mM Hepes buffer and 0.1% sodium azide. Aliquots of each batch were then stored at 4°C for routine use or −20°C for stocks.

Example 4. Preparation of monoclonal anti-A,B (A15/1A4.7)

Male B10 BR mice (OLAC) were immunised with $A_1$ cells followed by several injections of human blood group A substance. Spleen cells ($10^8$) from the immunised mice were fused with mouse myeloma cells ($10^7$) by the use of the polyethylene glycol method. The myeloma cells were non-secretor NS0/1 derived from P3-NS1/1/Ag4/1 selected enzyme deficient cells that die in HAT medium.

The culture supernatants were tested, beginning 2 week post fusion and those cells producing anti-A,B were selected by agglutination tests, then cloned on soft agar, grown to larger volumes, recloned, regrown and divided into aliquots for larger scale antibody production and liquid nitrogen storage of the selected cell lines.

To prepare culture supernatants for testing, the established anti-A,B secreting clone (A15/1A4.7) was grown in Dulbecco's modified Eagle's medium (DMEM, Gibco Biocult), supplemented with foetal calf serum (FCS, Serolab). The concentration of FCS was gradually reduced from 15% to 2.5%.

The supplemented tissue culture medium was free of anti-A or anti-B activity and A or B substances.

5

Example 5. Blending monoclonal anti-A and anti-B reagents.

An ABO blood cell grouping reagent was prepared by mixing (in a 1:1:1 (v/v/v) ratio) monoclonal anti-A supernatant (5 fold concentrate, Example 1), monoclonal anti-B supernatant (Example 3) and group O serum (from a Hyperimmunised group O donor). Titration scores and avidity times for this mixture were compared with those of a group O serum obtained from a hyperimmunised group O donor (and in the latter case with commercial and NHS group O sera). Results are given in Tables 1 and 2.

TABLE 1
Blending monoclonal anti-A, anti-B and group O serum-titrations

Equal parts anti-A 6D4×5+anti-B NB1/19+group O serum

| 2% cells | | | | | | Dilutions | | | | | | |
|---|---|---|---|---|---|---|---|---|---|---|---|---|
| | 1 | 2 | 4 | 8 | 16 | 32 | 64 | 128 | 256 | 512 | 1024 | 2048 |
| $A_1$ | C | C | C | C | +++ | ++ | + | (+) | (+) | GW | GW | — |
| $A_2$ | C | C | +++ | ++ | + | (+) | (+) | GW | GW | — | — | — |
| $B_3$ | C | C | C | +++ | + | + | (+) | GW | GW | W | W | — |
| $A_3$ | + | + | + | + | (+) | (+) | GW | W | — | Free cell picture | | |
| $A_x$ | W | W | W | W | W | — | — | — | — | — | — | — |
| O | — | — | — | — | — | — | — | — | — | — | — | — |

| | | | | | | Group O serum | | | | | |
|---|---|---|---|---|---|---|---|---|---|---|
| | 1 | 2 | 4 | 8 | 16 | 32 | 64 | 128 | 256 | 512 |
| $A_1$ | C | C | C | +++ | ++ | + | GW | W | W | — |
| $A_2^-$ | C | +++ | +++ | ++ | + | + | GW | — | — | — |
| B | C | C | C | +++ | +++ | + | (+) | GW | W | — |
| $A_3$ | + | + | (+) | (+) | (+) | GW | W | — | Free cell picture | |
| $A_x$ | GW (+) | GW | W | W | W | — | — | — | — | — |
| O | — | — | — | — | — | — | — | — | — | — |

NB   C=Complete
GW=Good/Weak, requires microscope to determine
W=Weak

TABLE 2
Blending monoclonal anti-A, anti-B and group O serum-avidity

| Anti-A+Anti-B reagent | Avidity tests (s) ×20% cells | | |
|---|---|---|---|
| | $A_2$ | B | O control |
| Commercial | 5 | 5 | 0 |
| NHS (1750) | 11 | 12 | 0 |
| Hyperimmunised Group O serum | 10 | 11 | 0 |
| Blend 6D4×5+NB1/19+Group O serum, equal volumes | 9 | 10 | 0 |

Example 6. Blending monoclonal anti-A and anti-B reagents

An ABO blood cell grouping reagent was prepared by mixing (in a 1:1 (v/v) ratio) monoclonal anti-A supernatant (2 fold concentrate, Example 2) and monoclonal anti-B supernatant (2 fold concentrate, Example 3). Titration scores for this mixture are given in Table 3. The avidity scores for the mixture, which were compared to commercial sera, are given in Table 4.

TABLE 3

1) Equal parts anti-A 3D3×2+anti-B NB1/19×2

| 2% cells | Dilutions | | | | | | | | | |
|---|---|---|---|---|---|---|---|---|---|---|
| | 1 | 2 | 4 | 8 | 16 | 32 | 64 | 128 | 256 | 512 |
| $A_1$ | C | C | +++ | +++ | ++ | ++ | + | (+) | (+) | GW |
| $A_2$ | C | C | +++ | +++ | ++ | + | + | (+) | GW | GW |
| $A_1B$ | C | C | C | C | ++ | + | + | + | (+) | (+) |
| $A_2B$ | C | C | +++ | +++ | ++ | + | + | + | (+) | GW |
| B | C | C | +++ | +++ | ++ | + | + | + | (+) | W |
| $A_3$ | ++ | + | + | (+) | (+) | (+) | GW | W | W | Free cell picture |
| $A_x$ | — | — | — | — | — | — | — | — | — | — |
| O | — | — | — | — | — | — | — | — | — | — |

TABLE 4

| Anti-A/Anti-B reagents | Avidity tests (s) ×20% cells | | |
|---|---|---|---|
| | $A_2$ | B | O control |
| Commercial | 5 | 5 | 0 |
| Blend 3D3×2+NB1/19×2 equal volumes | 4 | 5 | 0 |

Agglutination reactions were all 4+ after two minutes with $A_2$ and B cells.

Example 7. Blending monoclonal anti-A, anti-B and anti-A,B reagents

An ABO blood cell grouping reagent was prepared by mixing (in a 1:1:1 (v/v/v) ratio) monoclonal anti-A supernatant (two fold concentrate, Example 2), monoclonal anti-B supernatant (two fold concentrate, Example 3) and monoclonal anti-A,B supernatant (Example 4). Titration scores for this mixture are give in Table 5. The avidity scores for the mixture, which were compared to commercial sera, are given in Table 6.

TABLE 5

| 2) Equal parts anti-A 3D3×2+anti-B NB1/19×2+anti-A,B A15/1A4.7 | | | | | | | | | |
|---|---|---|---|---|---|---|---|---|---|
| 1 | 2 | 4 | 8 | 16 | 32 | 64 | 128 | 256 | 512 |
| A₁ | | | | | | | | | |
| C | C | +++ | +++ | ++ | ++ | + | + | + | (+) |

| | 1 | 2 | 4 | 8 | 16 | 32 | 64 | 128 | 256 | 512 |
|---|---|---|---|---|---|---|---|---|---|---|
| A₁ | C | C | +++ | +++ | ++ | ++ | + | + | + | (+) |
| A₂ | C | C | +++ | +++ | ++ | ++ | + | + | (+) | W |
| A₁B | C | C | C | +++ | +++ | ++ | + | + | (+) | W |
| A₂B | C | C | +++ | +++ | ++ | + | + | + | (+) | GW |
| B | C | C | +++ | +++ | ++ | ++ | + | (+) | GW | — |
| A₃ | ++ | + | + | (+) | (+) | GW | GW | W | Free cell picture | |
| Aₓ | — | — | — | — | — | — | — | — | — | — |
| O | — | — | — | — | — | — | — | — | — | — |

TABLE 6

| Anti-A/Anti-B reagents | Avidity tests (s) ×20% cells | | |
|---|---|---|---|
| | A | B | O control |
| Commercial | 5 | 5 | 0 |
| Blend 3D3×2+NB1/19+A15/1A4.7 equal volumes | 4 | 6 | 0 |

Agglutination reactions were all 4+ after two minutes with A₂ and B cells.

**Claims for the Contracting States: BE, CH, DE, FR, LI, LU, NL, SE**

1. An ABO blood grouping reagent comprising, in admixture, one or more anti-A monoclonal antibodies and one or more anti-B monoclonal antibodies.

2. An ABO blood grouping reagent according to claim 1 wherein one or more of the monoclonal antibodies has Ax activity.

3. An ABO blood grouping reagent according to claim 1 wherein one or more of the monoclonal antibodies has Bx activity.

4. An ABO blood grouping reagent according to claim 1 wherein the one or more monoclonal antibodies are derived from hybrid myelomas prepared by fusion of spleen cells with mouse myeloma cells selected from NSI cells and NSO cells.

5. An ABO blood grouping reagent according to claim 1 further comprising Group O serum.

6. A process for the grouping of ABO blood cells comprising adding to a suspension of cells in sodium chloride solution a blood cell grouping reagent according to claim 1 and detecting the agglutination or non-agglutination of the cells within the solution.

7. A blood cell grouping kit for use in the process of claim 6 comprising an ABO blood grouping reagent according to claim 1.

8. A blood cell grouping kit for use in the process of claim 6 comprising, in separate containers,
(a) one or more anti-A monoclonal antibodies, and
(b) one or more anti-B monoclonal antibodies.

9. A blood cell grouping kit according to claim 8 further comprising Group O serum.

**Claims for the Contracting State: AT**

1. A process for preparing an ABO blood grouping reagent which comprises mixing one or more anti-A monoclonal antibodies together with one or more anti-B monoclonal antibodies.

8

2. A process according to claim 1 wherein one or more of the monoclonal antibodies has Ax activity.

3. A process according to claim 1 wherein one or more of the monoclonal antibodies has Bx activity.

4. A process according to claim 1 wherein the one or more monoclonal antibodies are derived from hybrid myelomas prepared by fusion of spleen cells with mouse myeloma cells selected from NSI cells and NSO cells.

5. A process according to claim 1 further comprising mixing Group O serum together with the monoclonal antibodies.

6. A process for the grouping of ABO blood cells comprising adding to a suspension of cells in sodium chloride solution a blood cell grouping reagent which is the product of the process according to claim 1, and detecting the agglutination or non-agglutination of the cells within the solution.

7. A blood cell grouping kit for use in the process of claim 6 comprising the product of the process according to claim 1.

8. A blood cell grouping kit for use in the process of claim 6 comprising, in separate containers,
(a) one or more anti-A monoclonal antibodies, and
(b) one or more anti-B monoclonal antibodies.

9. A blood cell grouping kit according to claim 8 further comprising Group O serum.

**Patentansprüche für die Vertragsstaaten: BE, CH, DE, FR, LI, LU, NL, SE**

1. ABO-Blutgruppenbestimmungsreagens, gekennzeichnet durch ein Gemisch mindestens eines monclonalen Anti-A-Antikörpers und mindestens eines monclonalen Anti-B-Antikörpers.

2. ABO-Blutgruppenbestimmungsreagens nach Anspruch 1, dadurch gekennzeichnet, daß mindestens einer der monoclonalen Antikörper Ax-Aktivität aufweist.

3. ABO-Blutgruppenbestimmungsreagens nach Anspruch 1, dadurch gekennzeichnet, daß mindestens einer der monoclonalen Antikörper Bx-Aktivität aufweist.

4. ABO-Blutgruppenbestimmungsreagens nach Anspruch 1, dadurch gekennzeichnet, daß die monclonalen Antikörper von Hybridmyeloma abstammen, die durch Fusion von Milzzellen mit Mäusemyelomazellen, ausgewählt unter NSI- und NSO-Zellen, erhalten worden sind.

5. ABO-Blutgruppenbestimmungsreagens nach Anspruch 1, gekennzeichnet ferner durch ein O-Gruppenserum.

6. Verfahren zur Bestimmung der ABO-Blutgruppenzellen, gekennzeichnet durch Zugabe eines ABO-Blutgruppenbestimmungsreagens gemäß Anspruch 1 zu einer Zellsupsension in Natriumchlorid und Nachweis des Auftreten oder Nichtauftreten von Agglutination in der Lösung.

7. Blutgruppenbestimmungsreagens-Packung (Kit) zur Verwendung im Verfahren gemäß Anspruch 6, gekennzeichnet durch ein ABO-Blutgruppenreagens gemäß Anspruch 1.

8. Blutgruppenbestimmungsreagens-Packung zur Verwendung im Verfahren gemäß Anspruch 6, gekennzeichnet durch
(a) mindestens einen monoclonalen Anti-A-Antikörper und
(b) mindestens einen monoclonalen Anti-B-Antikörper in getrennten Behältern.

9. Blutgruppenbestimmungsreagens-Packung nach Anspruch 8, gekennzeichnet ferner durch ein O-Gruppenserum.

**Patentansprüche für den Vertragsstaat: AT**

1. Verfahren zur Herstellung eines ABO-Blutgruppenbestimmungsreagens, dadurch gekennzeichnet, daß man mindestens einem monoclonalen Anti-A-Antikörper mit mindestens einem monoclonalen Anti-B-Antikörper vermischt.

2. Verfahren nach Anspruch 1, dadurch gekennzeichnet, daß mindestens einer der monoclonalen Antikörper Ax-Aktivität aufweist.

3. Verfahren nach Anspruch 1, dadurch gekennzeichnet, daß mindestens einer der monoclonalen Antikörper Bx-Aktivität aufweist.

4. Verfahren nach Anspruch 1, dadurch gekennzeichnet, daß die monoclonalen Antikörper von Hybridmyeloma abstammen, die durch Fusion von Milzzellen mit Mäusemyelomazellen, ausgewählt unter NSI- und NSO-Zellen, erhalten worden sind.

5. Verfahren nach Anspruch 1, dadurch gekennzeichnet, daß weiters ein O-Gruppenserum mit den monoclonalen Antikörpern vermischt wird.

6. Verfahren zur Bestimmung der ABO-Blutgruppenzellen, gekennzeichnet durch Zugabe eines Blutgruppenzellenreagens, hergestellt nach dem Verfahren gemäß Anspruch 1 zu einer Zellsuspension in Natriumchlorid und Nachweis des Auftretens oder Nichtauftretens von Agglutination der Zellen in der Lösung.

7. Blutzellengruppenbestimmungs-Packung zur Verwendung im Verfahren gemäß Anspruch 6, gekennzeichnet durch ein ABO-Blutgruppenreagens gemäß Anspruch 1.

8. Blutzellengruppenbestimmungs-Packung zur Verwendung im Verfahren gemäß Anspruch 6, gekennzeichnet durch
(a) mindestes einen monoclonalen Anti-A-Antikörper und
(b) mindestens einen monoclonalen Anti-B-Antikörper in getrennten Behältern.

9. Blutgruppenbestimmungsreagens-Packung nach Anspruch 8, gekennzeichnet ferner durch ein O-Gruppenserum.

**Revendications pour les Etats Contractants: BE, CH, DE, FR, LI, LU, NL, SE**

1. Réaction pour la détermination du groupe sanguin dans le système ABO, comprenant, en mélange, un ou plusieurs anticorps monoclonaux anti-A et un ou plusieurs anticorps monoclonaux anti-B.

2. Réactif pour la détermination du groupe sanguin dans le système ABO, selon la revendication 1, dans lequel ou un plusieurs de anticorps monoclonaux possèdent de l'activité Ax.

3. Réactif de détermination du groupe sanguin dans le système ABO, selon la revendication 1, dans lequel un ou plusieurs des anticorps monoclonaux possèdent de l'activité Bx.

4. Réactif de détermination du groupe sanguin dans le système ABO, selon la revendication 1, dans lequel un ou plusieurs des anticorps monoclonaux dérivent de myélomes hybrides préparés par fusion de cellules de rate avec des cellules de myélomes de souris choisies parmi des cellules NSI et des cellules NSO.

5. Réactif de détermination du groupe sanguin dans le système ABO, selon la revendication 1, comprenant en outre du sérum de groupe O.

6. Procédé pour la détermination du groupe de cellules ou globules sanguins, dans le système ABO, comprenant l'addition, à une suspension des globules dans une solution de chlorure de sodium, d'un réactif pour détermination de groupes de globules sanguins selon la revendication 1, et la détection de l'agglutination ou de la non-agglutination des cellules dans la solution.

7. Trousse pour la détermination du groupe de globules de sang, destinée à servir dans le procédé de la revendication 6, comprenant un réactif, selon la revendication 1, pour la détermination du groupe sanguin dans le système ABO.

8. Trousse pour la détermination du groupe de globules de sang, destinée à servir dans le procédé de la revendication 6, comprenant, dans des récipients séparés,
   a) un ou plusieurs anticorps monoclonaux anti-A, et
   b) un ou plusieurs anticorps monoclonaux anti-B

9. Trousse pour la détermination du groupe de globules de sang selon la revendication 8, comprenant en outre du sérum de groupe O.

**Revendications pour l'Etat Contractant: AT**

1. Procédé pour la préparation d'un réactif de détermination du groupe sanguin dans le système ABO, procédé qui comprend le mélangeage d'un ou plusieurs anticorps monoclonaux anti-A et d'un ou plusieurs anticorps monoclonaux anti-B.

2. Procédé selon la revendication 1, dans lequel un ou plusieurs des anticorps monoclonaux possèdent de l'activité Ax.

3. Procédé selon la revendication 1, dans lequel un ou plusieurs des anticorps monoclonaux possèdent de l'activité Bx.

4. Procédé selon la revendication 1, dans lequel un ou plusieurs des anticorps monoclonaux dérivent de myélomes hybrides préparés par fusion de cellules de rate avec des cellules de myélomes de souris choisies parmi des cellules NSI et des cellules NSO.

5. Procédé selon la revendication 1, comprenant en outre le mélangeage du sérum de groupe O avec les anticorps monoclonaux.

6. Procédé pour la détermination du groupe de cellules ou globules sanguins, dans le système ABO, comprenant l'addition, à une suspension des globules dans une solution de chlorure de sodium, d'un réactif pour détermination de groupes de globules sanguins, qui est le produit du procédé selon la revendication 1, et la détection de l'agglutination ou de la non-agglutination des cellules dans la solution.

7. Trousse pour la détermination du groupe de globules de sang, destinée à servir dans le procédé de la revendication 6, comprenant le produit du procédé selon la revendication 1.

8. Trousse pour la détermination du groupe de globules de sang, destinée à servir dans le procédé de la revendication 6, comprenant, dans des récipients séparés,
   a) un ou plusieurs anticorps monoclonaux anti-A, et
   b) un ou plusieurs anticorps monoclonaux anti-B

9. Trousse pour la détermination du groupe de globules de sang selon la revendication 8, comprenant en outre du sérum de groupe O.